# EUROPEAN PATENT SPECIFICATION

(11) **EP 3 335 578 B1**
(45) Date of publication and mention of the grant of the patent: **28.04.2021**
(21) Application number: 17207549.1
(22) Date of filing: 15.12.2017
(51) Int. Cl.: A61K 36/899, A23L 33/145, A23L 31/10, A61K 36/062, C12P 1/02, A23L 7/104

(54) **RED RICE EXTRACTS STANDARDISED IN TOTAL MONACOLINS AND INDUSTRIAL PROCESSES FOR THE PRODUCTION THEREOF**
ROTE REISEXTRAKTE, DIE IN GESAMTMONACOLINEN STANDARDISIERT SIND UND INDUSTRIELLEN PROZESSEN ZUR HERSTELLUNG.
EXTRAITS DE RIZ ROUGES NORMALISÉS EN MONACOLINES TOTALES ET PROCÉDÉS INDUSTRIELS POUR LA PRODUCTION DE CEUX-CI.

(30) Priority: 16.12.2016 IT 201600127414
(43) Date of publication of application: 20.06.2018
(73) Proprietor: Labiotre S.r.l., 50028 Tavarnelle Val di Pesa FI (IT)
(72) Inventor: Nannoni, Giulia, 50028 Tavarnelle Val di Pesa FI (IT); Alì, Alessandro, 50028 Tavarnelle Val di Pesa FI (IT)
(74) Representative: Perani & Partners S.p.A.

(56) References cited:
- EP-A1- 3 093 052
- CN-B- 102 432 573
- KR-A- 20130 090 268
- US-A1- 2010 278 983
- ANURAK BUNNOY ET AL: "Monascus purpureus-fermented Thai glutinous rice reduces blood and hepatic cholesterol and hepatic steatosis concentrations in diet-induced hypercholesterolemic rats", BMC COMPLEMENTARY AND ALTERNATIVE MEDICINE, BIOMED CENTRAL LTD., LONDON, GB, vol. 15, no. 1, 28 March 2015 (2015-03-28) , page 88, XP021220371, ISSN: 1472-6882, DOI: 10.1186/S12906-015-0624-5
- CHUN-LIN LEE ET AL: "Red mold rice extract represses amyloid beta peptide-induced neurotoxicity via potent synergism of anti-inflammatory and antioxidative effect", APPLIED MICROBIOLOGY AND BIOTECHNOLOGY, SPRINGER, BERLIN, DE, vol. 79, no. 5, 26 April 2008 (2008-04-26) , pages 829-841, XP019623633, ISSN: 1432-0614
- Wei Wei ET AL: "Hypolipidemic and anti-atherogenic effects of long-term Cholestin (Monascus purpureus-fermented rice, red yeast rice) in cholesterol fed rabbits", The Journal of Nutritional Biochemistry, vol. 14, no. 6, 1 June 2003 (2003-06-01), pages 314-318, XP055568292, AMSTERDAM, NL ISSN: 0955-2863, DOI: 10.1016/S0955-2863(03)00051-2

## Description

### FIELD OF THE INVENTION

The present invention relates to dried extracts of red rice fermented by Monascus purpureus, as well as the preparation processes of said dried extracts.

### PRIOR ART

Red yeast rice is a product originating in Chinese medicine, made from rice fermented by a particular yeast, *Monascus purpureus* or red yeast.

During the fermentation, said yeast is enriched with a group of 14 substances, called monacolins. Among these is monacolin K, which mirrors the chemical structure and pharmacological action of lovastatin, a drug which is part of the group of statins and dedicated to the treatment of dyslipidemia and metabolic syndromes.

Like lovastin, monacolin K acts positively on the systemic circulation, reducing cholesterol levels by inhibiting HMG-CoA reductase, a key enzyme in cholesterol biosynthesis.

The use of Chinese red yeast rice has increased exponentially in recent years due to the interest of the Western public to alternative and complementary medicines.

This product is an alternative to the lipid-lowering lovastatin-based drug therapy in those patients who do not want to take statins for philosophical reasons or who have liver or muscle intolerance to statins.

However, there are problems of standardization of the title in total monacolins of products containing red yeast rice extract, making it difficult to ensure the purity and content of the product itself, as well as the origin of monacolins contained therein. http://www.infomedicaintegrata.it/index.php/fitoterapia/riso-rosso-fermentato/riso-rosso-fermentato-la-statina-naturale-con-problemi-di-standardizzazione/

Sometimes, in fact, in order to increase the total monacolin title, the extracts are supplemented with active ingredients produced by synthesis or by fermentation of biofermentative starters other than Monascus purpureus.

Standardised extracts of red rice fermented by Monascus purpureus present on the world market are mostly manufactured in China and declare a content in total monacolins which ranges between 1.5% and 5%.

Chu-Lin Lee et al., Applied microbiology and biotechnology, 2008, vol. 79, no 5, 829-841, provides a red rice extract for use in the treatment of hypercholesterolemia and a method to obtain the extract by extraction with 95% ethanol at 37°C for 24 hours, to be combined with the red rice powder in a weight ratio of 1:50. The extract obtained has a monacolin K title of about 1 % by weight.

### SUMMARY OF THE INVENTION

The applicant has now found that standardised extracts of fermented red rice having a title of total monacolins (as monacolin K) higher than 5% can be made, wherein the total monacolins contained therein are 100% natural.

The present invention therefore relates to dry extracts of red rice fermented by Monascus purpureus having a title of total monacolins (as monacolin K) higher than 5%, wherein said total monacolins are 100% natural and are totally derived from the fermentation of rice by Monascus purpureus.

Also disclosed are dietary supplements containing, as dry extract, red rice fermented by Monascus Purpureus, the dry extract according to the present invention.

A further object of the present invention is the method of production of the dry extracts of the fermented red rice characterized by the above specifications.

### DESCRIPTION OF THE FIGURES

Figure 1: Block diagram representing one of the preferred embodiments of the method object of the present patent application. The figure shows the steps necessary for obtaining a dry extract of red rice with monacolins > 5%, starting from the first extract **a.** The dashed box in which the solid phase **c** is represented indicates that said solid phase of the second extract **c** is eliminated, to retain instead the liquid phase **d** of the second extract.

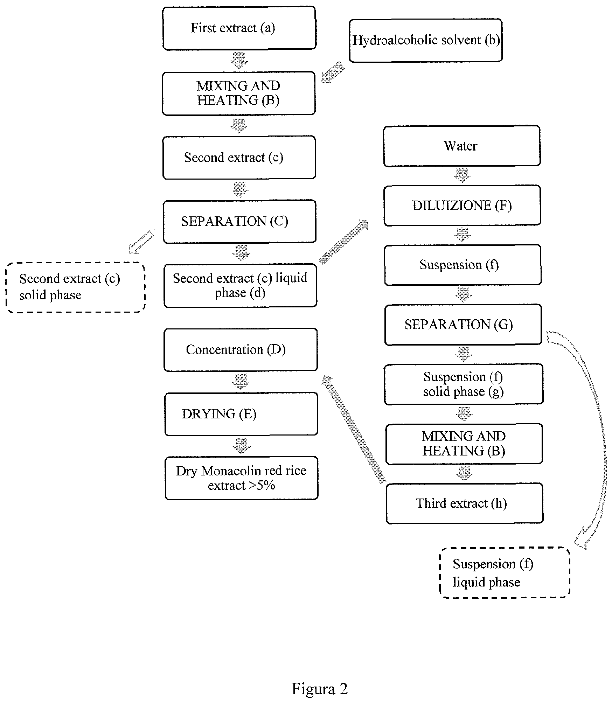

Figure 2: Block diagram representing a second embodiment of the method of interest. Figure 2 shows the steps of the method according to figure 1 integrated with further intermediate steps between the separation step C and the concentration step D.
   From the separation G of the suspension **f** a liquid phase of the suspension **f** and a solid phase **g** of the same are obtained, where said liquid phase of the suspension **f is** discarded.

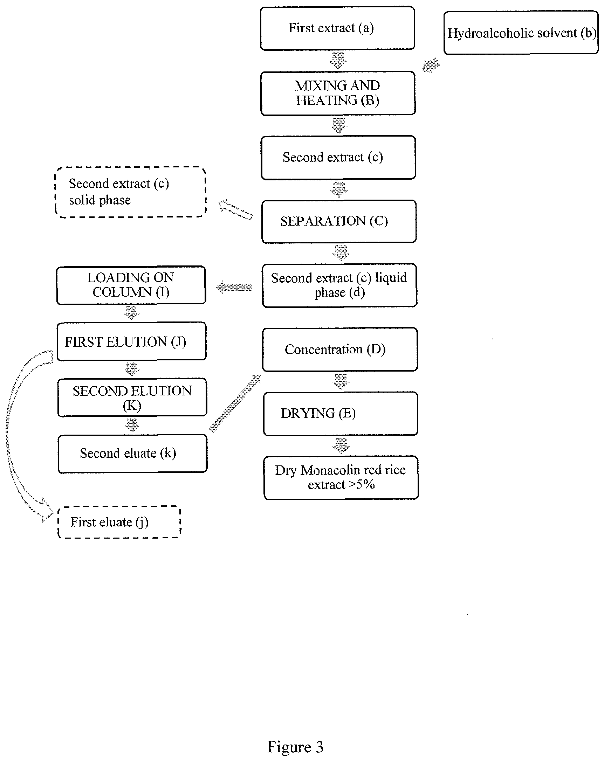

Figure 3: Block diagram representing a third embodiment of the method according to the invention. The figure shows the steps of the method according to figure 1 integrated with further intermediate steps between the separation step C and the concentration step D. The first eluate j, obtained by primary elution J with water from the separation column, is eliminated as not of interest for the production of the dry extract object of the invention.

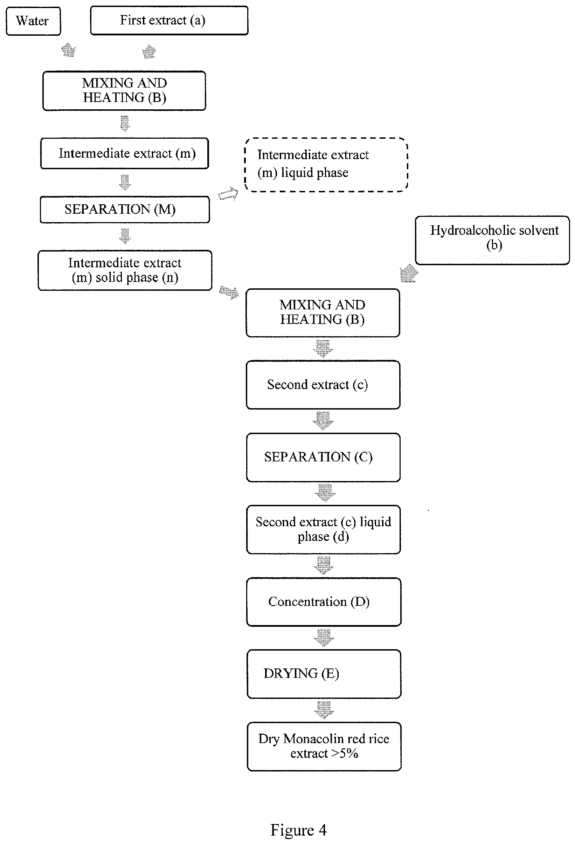

Figure 4: Block diagram representing a fourth embodiment of the method according to the invention. In this case, the method according to figure 1 provides that, before mixing and heating B, the first extract **a** is subjected to further sample preparation steps. From the separation M of the intermediate extract **m,** a liquid phase of the intermediate extract **m** and a solid phase **n** of the same are obtained, where only the second one is stored to be subjected to the subsequent steps of the method for preparing the dry red rice extract.
Figure 5: Fingerprint chromatogram of a fermented red rice extract from literature; MK = monacolin K (lactone form); MKA = monacolin K (hydroxy-acid form); DMK = dehydromonacolin K; MJA, MJ, MXA, MLA, MX, ML, P1, MMA, MM = other monacolins
Figure 6: Chromatogram of a commercial extract of 3% fermented red rice in total monacolins (adulterated), comprising almost exclusively monacolin K (extract 1).
Figure 7: Chromatogram of an extract of 1.5% fermented red rice in total monacolins. The extract corresponds to the starting extract (raw material) used in the method according to the present invention (extract 2).
Figure 8: Chromatogram of an extract of 7% fermented red rice in total monacolins. The extract corresponds to that obtained with the method according to the patent application (extract 3).
Figure 9: Granulate of the dry extract of fermented red rice according to the invention (extract 3).
Figure 10: Granulate of a commercial dry extract of fermented red rice (extract 1).

### DETAILED DESCRIPTION OF THE INVENTION

For the purposes of the present invention, dry extract means a solid preparation obtained by extraction and subsequent evaporation of the solvent used during the extraction.
By dry extract it is also meant an extract having a dry residue of not less than 90% by mass.

Soft extract means a preparation having an intermediate consistency between the fluid extracts and the dry extracts, obtained by evaporation of the solvent used for their preparation and normally characterized by a dry residue of not less than 60% by mass.

The dry extract object of the present invention advantageously contains 100% natural monacolins (such as monacolin K), and totally obtained by fermentation of the *Oryza Sativa* rice, preferably non-glutinous, by Monascus purpureus.
The total monacolins contained in the fermented red rice are varied. The title in total monacolins represents the sum of each of them, but is conventionally expressed as monacolin K.
Monacolin K (lactic and acidic) is preponderant and represents about 70-80% of the total.

Advantageously, the fermentation by Monascus purpureus produces, in addition to the monacolins themselves, also other active substances such as phytosterols, fatty acids (both mono- and poly-unsaturated) and isoflavones which contribute to the beneficial effects elicited in vivo by the fermented red rice extract.

Said further active substances would be lost or diluted if the dried red rice extract was not produced by fermentation by Monascus purpureus or supplemented with synthesis monocolins or other biofermentative route.

The title of total monacolins (as monacolin K) of the dry extract of fermented red rice described therein is more than 5% by weight of the total weight of the dry extract of fermented red rice (w/w).

Preferably, the total monacolin content (as monacolin K) is greater than 5% (w/w) and less than or equal to 80% (w/w) and even more preferably greater than 5% (w/w) and less than or equal to 20% (w/w).

Also disclosed are dietary supplements, comprising dry extract of red rice fermented by Monascus Purpureus wherein said red rice extract fermented by Monascus Purpureus consists exclusively of the dry red rice extract according to the present invention.

The dietary supplements are preferably formulated in the form of powders, granules and oral liquid pharmaceutical forms, according to the techniques known in the art and described in Remington, The Science and Practice of Pharmacy, 21st Edition.

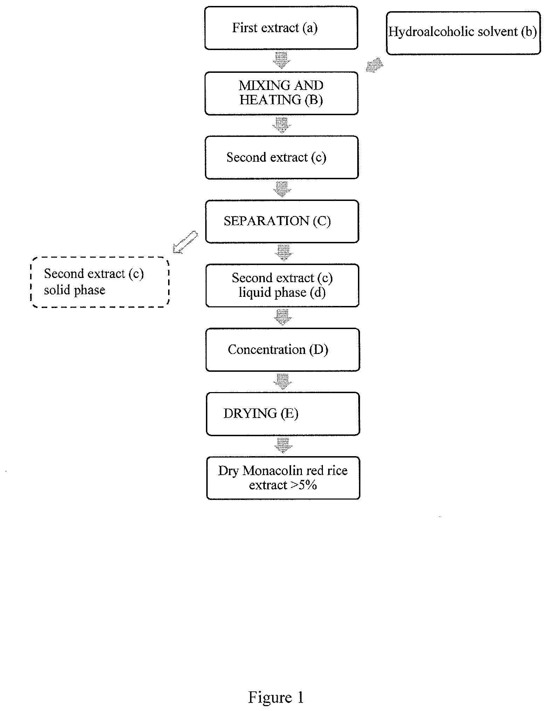

More preferably, said dietary supplements are used in the treatment of hypercholesterolemia, due to the inhibitory effect that monacolins exhibit towards the synthesis of endogenous cholesterol.
A further object of the present patent application is a method for producing the dry extract of red rice fermented by Monascus purpureus according to the invention.
The method allows producing standardized dried extracts of fermented red rice with a title of total monacolins (as monacolins K) greater than 5%, advantageously without adding, in any step of the method, synthetic monacolins or derived from the fermentation of biofermentative starters other than Monascus purpureus, such as Aspergillus.
Advantageously, said method of production, in all the embodiments thereof, is the only method responsible for obtaining the title in total monacolins contained in the dry extract of red rice fermented by Monascus purpureus, object of the invention. With reference to the accompanying Figure 1, the main steps of the process object of the present patent application are schematically represented in a flow chart, where first extract **a** indicates a first dry extract of red rice fermented by Monascus purpureus with a title in total monacolins (as monacolin K) less than or equal to 3.30% and wherein said monacolins are derived exclusively and completely by fermentation of red rice by Monascus purpureus.

In the preferred embodiment, the method object of the present invention provides that, once the first extract **a** (step A) has been arranged, said first extract is subjected to the subsequent steps of:
B. Mixing and heating the first extract **a** with a hydroalcoholic solvent **b,** in a weight ratio of between 1:14 and 1:22, in which the heating is carried out at a temperature of between 50° and 70 °C, for a time of between 40 and 80 minutes, under stirring, so as to obtain a second extract **c.**
C. Isolating, by separation, of a liquid phase **d** of said second extract **c** from a solid phase of said second extract **c.**
D. Concentrating D the liquid phase **d** of the second extract **c** under vacuum, at a temperature of 42-48 °C, until obtaining a soft extract **e** characterized by a dry residue at least equal to 20% by weight of the total weight of the soft extract (w/w).
E. Drying the soft extract **e,** until obtaining the dry extract of red rice object of the present patent application, fermented by Monascus purpureus and having a title of total monacolins (as monacolin K) of more than 5%.

The method object of the present invention provides that the hydroalcoholic solvent **b** is an ethanol/water mixture having an alcoholic content of between 30° and 96° alcoholic degrees.

According to the preferred embodiment, the ethanol/water mixture preferably has an alcoholic content of between 40° and 70° alcoholic degrees.

The separation step C can be carried out by any technique known to those skilled in the art, which allows separating a solid or a colloidal material from a liquid system.

By separation it is meant an operation that allows the isolation and/or purification of a single chemical constituent or a set of substances that can be assimilated from a chemical point of view, from a complex matrix of substances.

The method according to the invention provides, in all the embodiments thereof, that the separation is preferably carried out by clarification, decantation or centrifugation, and/or filtration of the substrate to be purified, according to the intrinsic properties of the system of interest.

The concentration step D can be carried out according to any technique known to the man skilled in the art, for example under vacuum with bubble evaporator, falling film or thin layer.

The drying step E is preferably carried out by means of a technique selected from the group consisting of spray drying, fluid bed granulator, drying cabinet, freeze drying, using a support selected for example from maltodextrin, arabic gum, inulin, vegetable fibres, polyalcohols, fructooligosaccharides.
Said support is dissolved in the substrate to be subjected to drying.

The dry extract obtained by the above embodiment of the method allows obtaining an extract with a maximum content of total monacolins (as monacolin K) of 15-25%.

The final title of the dry extract obtained by the method object of the present invention is preferably determined by quantitative techniques of high-pressure or high-performance liquid chromatography (HPLC).

In an alternative embodiment, following the separation C, the method according to the present invention preferably provides that the liquid phase **d** is subjected to the following steps:
F. Diluting with water the liquid phase **d** of the second extract **c** in order to obtain a suspension **f** characterized by an alcohol content of between 15° and 20° alcoholic degrees.
G. Isolating, by separation G, a liquid phase of suspension **f** from a solid phase **g** of said suspension **f.**
H. Mixing and heating H the solid phase **g** of suspension **f,** wherein said solid phase **g** is combined with the hydro-alcoholic solvent **b** in a weight ratio comprised between 1:14 and 1:22 and heating is carried out at a temperature comprised between 50° and 70°C, for a time comprised between 40 and 80 minutes, under stirring.

The mixing and heating H of the solid phase **g** of suspension **f** as described above allow obtaining a third extract **h.**

The extract **h,** alternatively to the liquid phase **d** of the second extract c, is subjected to the concentration phase D and to the drying phase E, in order to obtain the dry extract of fermented red rice object of the present invention.

The title of the dry extract obtained with said alternative embodiment of the method ranges from 15 to 25% of total monacolins (as monacolin K).

In a second alternative embodiment, the method according to the present invention preferably comprises, after the separation step C, the following treatment steps:
I. Loading of the liquid phase d of said second extract c on a column packed with adsorption resin.
J. Primary elution of the column with water and elimination of a first eluate **j,** resulting from said first elution.
K. Secondary elution of the column with ethanol and collection of a second eluate **k.** The ethanol used in this second elution step has an alcoholic content preferably of between 70° and 90° alcoholic degrees.

The second eluate **k,** collected from said second elution, is recovered in order to subject it to the concentration C and drying E step, alternatively to the liquid phase **d** of the second extract **c.**

From said concentration C and drying E, the dry extract of fermented red rice, object of the present patent application, is obtained.

In this second alternative embodiment, the column elution is preferably carried out by reversed phase distribution, employing a non-polar packing resin.

Preferably, both the primary elution J and the secondary elution K are conducted by recirculation and are possibly repeated several times with fresh solvent fractions.

In said second alternative embodiment, both the collection step of the first eluate **j** and of the second eluate **k** is preferably carried out until saturation of the eluting solvent.

The saturation of the elution solvent is preferably detected by quantitative HPLC techniques.

The second alternative embodiment advantageously allows obtaining a purified dry extract with a total monacolin title (as monacolin K) higher than 80%.

The method object of the present patent application can be alternatively implemented according to a third preferred embodiment, which provides a preliminary treatment of the first extract **a** according to the following steps:
L. Mixing and heating L of the first extract with water, wherein water and first extract **a** are mixed in a weight ratio of between 1:10 and 1:20, and heating is preferably carried out at a temperature of between 50° and 70 °C, for a time of between 40 and 80 minutes.

From the mixing and heating phase L, an intermediate extract **m** is obtained.

M. Isolation, by separation, of a liquid phase of said intermediate extract **m** and a solid phase **n** of the intermediate extract **m.**

In this third alternative embodiment, said solid phase **n** of the intermediate extract **m** is recovered and subjected, alternately to the first extract **a,** to the subsequent mixing and heating B, separation C, concentration D and drying E steps in order to obtain the dry extract of fermented red rice object of the present patent application.

The latter preferred embodiment advantageously allows obtaining a purified dry extract with a title in total monacolins (as monacolin K) comprised between 15-25%.

Advantageously, each of the embodiments of the method object of the present invention can be combined with the purification of column packed with adsorption resin, in order to obtain a total monacolin title (as monacolin K) higher than 80%.

Advantageously, the method object of the present patent application allows obtaining extracts of fermented red rice using physical purification methods, without involving chemical reactions, preserving and thus guaranteeing the naturalness of the product even after the purification process.

### EXAMPLES

The following are the results of chromatographic analyses carried out on samples of dried extract of fermented red rice present on the market and on the dry extract of fermented red rice object of the patent application.

### Experimental data

Monacolins are a group of compounds that exist in two forms, as lactone and as hydroxy-acid.

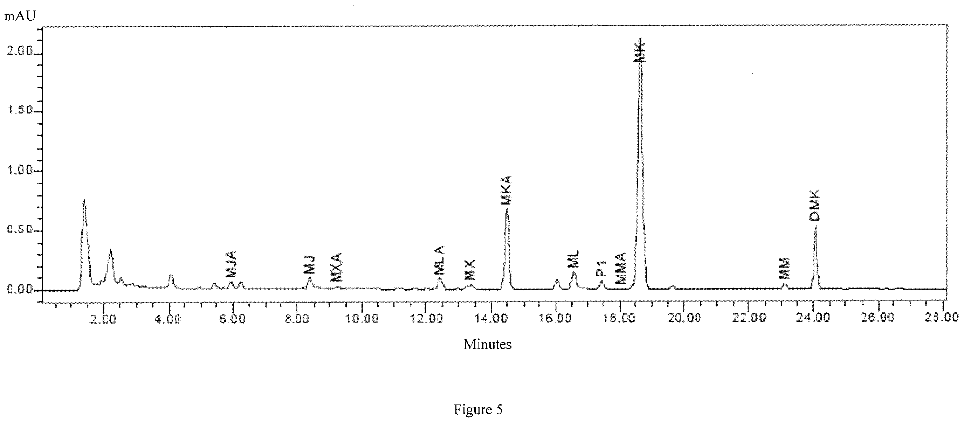

Figure 5 shows a typical fingerprint chromatogram of a fermented red rice extract found in the literature, where the peaks of
- monacolin K in lactone form (MK),
- monacolin K in hydroxy-acid form (MKA)
- monacolin K in the dehydrogenated form (DMK),
- other monacolins M present in the extract (MJA, MJ, MXA, MLA, MX, ML, PI, MMA, MM), are shown.

Currently on the market there are dried extracts of fermented red rice with titles equal to 5%, 3% and 1.5% of total monacolins expressed as monacolin K.

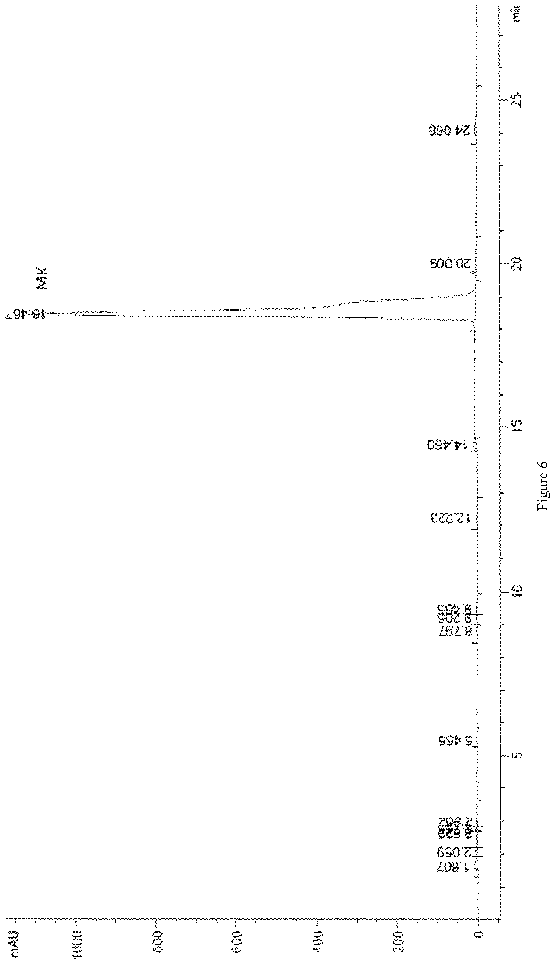

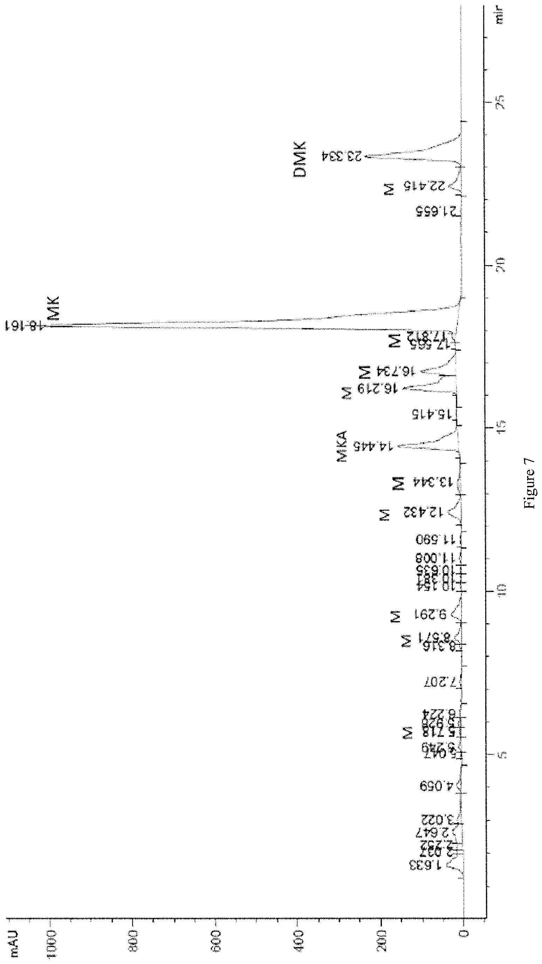

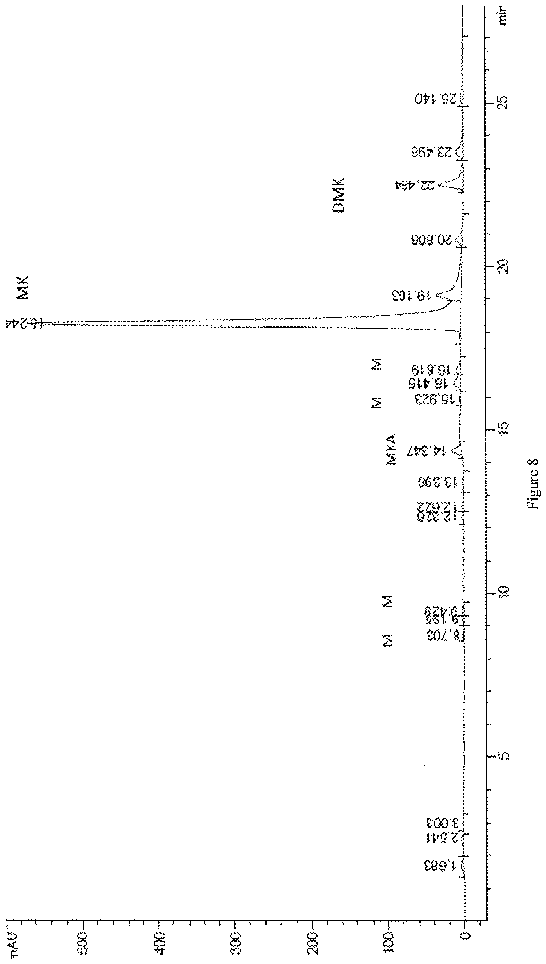

Figures 6, 7 and 8 show the results of the analyses carried out respectively on:
- Fermented red rice extract 3% Total monacolins (adulterated) - Extract 1;
- Fermented red rice extract 1.5% Total monacolins (raw material of the method object of the present patent application) - Extract 2;
- Fermented red rice extract 7% Total monacolins (purified product object of the patent application) - Extract 3.

The chromatograms were acquired at 237 nm, absorption wavelength of the monacolins.

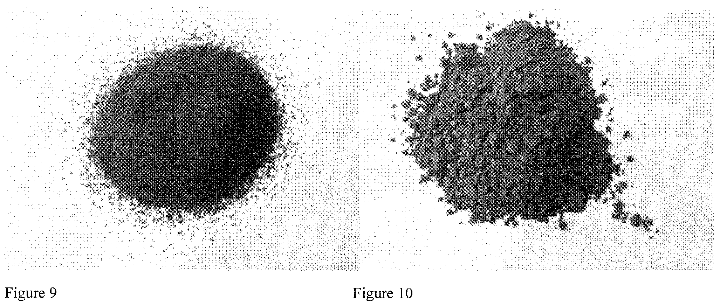

From the chromatograms in Figures 6, 7 and 8 it is observed that:
Extract 1 - there is the presence of monacolin K (MK) alone in lactone form. A fingerprint profile of this kind indicates a probable sophistication of the product by adding the active synthesis ingredient (lovastatin).
Extract 2 - various peaks corresponding to monacolins (M) and the main peaks of the 3 forms of monacolin K can be detected: hydroxy acid (MKA), lactone (MK), dehydromonacolin K (DMK). In this extract, the content of monacolin K is about half of the total monacolin content.
Extract 3 - the dry extract of fermented red rice obtained with the method according to the invention has a profile which denotes very well that, despite the increase in concentration in the extract of monacolin K in lactone form (MK), the other monacolins (MKA, DMK, M) also remain.
For illustrative purposes, Figures 9 and 10 respectively show the granulate obtained according to the method object of the invention and the granulate of a product available on the market.

### Comments

The comparison of the chromatograms shows a substantial difference in the composition of the commercial red rice extract and the one object of the present patent application.

The second one, having been obtained by purification of a dry extract of fermented red rice, totally natural, has the absorption peaks of monacolin K, in its lactone, hydroxy-acid and dehydrogenated form, as well as the peaks of the other monacolins present in the extract in lower quantities.

The chromatogram relating to the commercial red rice extract, on the other hand, has the only absorption peak of the lactonc-typc monacolin K.

This last chromatogram resembles the chromatogram of a pure substance (lovastatin/monacolin K), in which the peaks of monacolins M have almost disappeared against an increase in the concentration of monacolin K alone on the total extract.

The presence of only the absorption peak of monacolin K is probably indicative of the fact that the extract in question was obtained by adulteration and not by purification, a process that would have led to an increase in the relative concentration of monacolin K on the total weight on the extract and a reduction in the concentration of the other monacolins, which would have been retained in the purification process as impurities.

### EXAMPLE 1

Preparation of purified extracts of red rice fermented by Monascus purpureus with a title of total monacolins (as monacolin K) > 5% by solubilization in a suitable solvent.
A. Arrange 1000 g of dry extract of red rice fermented with a title of total monacolins (as monacolin K) equal to 3.20%.
B. Mix the above dry extract with 16,000 g of a mixture of ethanol-water 7-3 and heat at a temperature of 60 °C for 1 hour, under stirring;
C. Clarify the extract obtained by centrifugation and filter, recover the filtered liquid and discard the solid material;
D. Concentrate the clarified liquid under vacuum at a temperature of <45 °C until obtaining a soft extract with a dry residue of 20-30%;
E. Dry in a fluid bed granulator keeping the product temperature at <45 °C. Before the drying step, maltodextrin is added to the soft extract so as to standardize the title to the desired value, in the specific case 10% of the theoretical dry residue calculated on the soft extract.

The dry extract thus obtained has a title of total monacolins (as monacolin K) equal to 20.50%.

### EXAMPLE 2

Preparation of purified extracts of red rice fermented by Monascus purpureus with a title of total monacolins (as monacolin K) > 5% by precipitation and crystallization.
A. Arrange 1000 g of dry extract of red rice fermented with a title equal to 3.20% of total monacolins (as monacolin K);
**L.** Mix the above dry extract with 10,000 g of water and heat at a temperature of 60 °C for 1 hour, under stirring;
**M.** Clarify the extract obtained by centrifugation and filter, discard the filtered liquid and recover the solid residue;
B. Mix the above solid residue with a mixture of ethanol-water 7-3, in a ratio of 15 parts of hydroalcoholic mixture for each part of solid residue, and heat to a temperature of 60 °C for 1 hour, under stirring;
C. Filter the mixture above and recover the hydroalcoholic filtrate;
D. Concentrate the clarified liquid under vacuum at a temperature of <45 °C until obtaining a soft extract with a dry residue of 20-30%;
E. Dry in a fluid bed granulator keeping the product temperature at <45 °C. Before the drying step, maltodextrin is added as a carrier so as to standardize the title to the desired value, in the specific case 10% of the theoretical dry residue calculated on the soft extract.

The dry extract thus obtained has a title of total monacolins (as monacolin K) equal to 19.80%.

### EXAMPLE 3

Preparation of purified extracts of red rice fermented by Monascus purpureus with a title of total monacolins (as monacolin K) > 5% by solubilization and crystallization.
A. Arrange 1000 g of dry extract of red rice fermented with a title equal to 3.12 % of total monacolins (as monacolin K);
B. Mix the above dry extract with 16,000 g of a mixture of ethanol-water 7-3 and heat at a temperature of 60 °C for 1 hour, under stirring;
C. Clarify the extract obtained by centrifugation and filter, recover the filtered liquid and discard the solid material;
**F.** Dilute the filtered liquid with water until a final alcohol content of 10° is obtained;
**G.** Centrifuge, discard the clarified liquid and recover the solid material;
**H.** Mix the above dry extract with 16,000 g of a mixture of ethanol-water 7-3 and heat at a temperature of 60 °C for 1 hour, under stirring;
D. Concentrate the clarified liquid under vacuum at a temperature of <45 °C until obtaining a soft extract with a dry residue of 20-30%;
E. Dry in a fluid bed granulator keeping the product temperature at <45 °C. Before the drying step, maltodextrin is added as a carrier so as to standardize the title to the desired value, in the specific case 12 % of the theoretical dry residue calculated on the soft extract.
The dry extract thus obtained has a title of total monacolins (as monacolin K) equal to 18.70%.

### EXAMPLE 4

Preparation of purified extracts of red rice fermented by Monascus purpureus with a title of total monacolins (as monacolin K) > 5% by purification on resins.
A. Arrange 1000 g of dry extract of red rice fermented with a title equal to 3.05 % of total monacolins (as monacolin K);
B. Mix the above dry extract with 20,000 g of a mixture of ethanol-water 4-6 and heat at a temperature of 60 °C for 1 hour, under stirring;
C. Clarify the extract obtained by centrifugation and filter, recover the filtered liquid and discard the solid material;
**I.** Load the filtered liquid onto a column previously packed with adsorption resin styrene-divinylbenzene macroreticular copolymer, in a measure of 2 volumes of filtered liquid by volume of the resin bed, in which the column has dimensions equal to 70 cm height and 10 cm diameter.
**J.** Recirculate the column with a volume of water equal to 3 volumes of the resin bed (60 litres) and discard the eluate;
**K.** Recirculate the column with 120 kg of ethanol at 80° alcoholic degrees equal to about 6 volumes of the resin bed and recover the eluate when the solvent is saturated;
D. Concentrate the clarified liquid under vacuum at a temperature of <45 °C until obtaining a soft extract with a dry residue of 20-30%;
E. Dry in a fluid bed granulator keeping the product temperature at <45 °C. Before the drying step, maltodextrin is added as a carrier so as to standardize the title to the desired value, in the specific case 8 % of the theoretical dry residue calculated on the soft extract.
The dry extract thus obtained has a title of total monacolins (as monacolin K) equal to 23.60%.

### EXAMPLE 5 (not according to the invention)

Dietay supplement composition, in a tablet form.

| **Ingredient** | **Quantity** |
|---|---|
| D.E. Berberis Aristata 97% | 530 mg |
| D.E. Red yeast rice 20%: | 50 mg |
| D.E. Milk thistle 70% | 150 mg |
| Maltodextrin | 100 mg |
| Cross-linked sodium carboxy methyl cellulose | 100 mg |
| Cellulose | 150 mg |
| Stearic acid | 50 mg |
| Magnesium stearate | 30 mg |
| Silicon dioxide | 40 mg |
| **TOTAL** | 1200 mg |

| | |
|---|---|
| Total monacolin content (as monacolin K): 10 mg per tablet | |

## Claims

1. Method for the production of a dry extract of red rice fermented by Monascus purpureus having a title of total monacolins, expressed as monacolin K, higher than 5% and lower than or equal to 80%, wherein said total monacolins are 100% natural and totally obtained by fermentation of rice by Monascus purpureus, the method comprising the steps of:
A. Arranging a first dry extract (a) of red rice fermented by Monascus purpureus with a title of total monacolins, expressed as monacolin K, lower than or equal to 3.30%,
B. Mixing said first extract (a) with a hydro-alcoholic solvent (b) in a weight ratio comprised between 1:14 and 1:22; heating at a temperature comprised between 50° and 70°C, for a time comprised between 40 and 80 minutes, under stirring and obtaining a second extract (c),
C. Isolating, by separation, of a liquid phase (d) of said second extract (c) from a solid phase of said second extract (c),
D. Concentrating said liquid phase (d) of the second extract (c) under vacuum at a temperature lower than 45°C, until obtaining a soft extract (e) with a dry residue at least equal to 20%,
E. Drying said soft extract (e) until reaching the desired title t, and **characterised in that**
said alcoholic solvent (b) is an ethanol/water mixture having an alcoholic content comprised between 30° and 96° alcoholic degrees.

2. Method according to claim 1, wherein the isolation step C further comprises, before step D, the steps of:
F. Diluting the liquid phase (d) of the second extract (c) with water and obtaining a suspension (f) of the liquid phase (d) of the second extract (c) with an alcohol content comprised between 15° and 20°,
G. Isolating, by separation, a liquid phase of said suspension (f) from a solid phase (g) of said suspension (f),
H. Mixing the solid phase (g) of the suspension (f) of the liquid phase (d) of the second extract (c) with the hydro-alcoholic solvent (b) in a weight ratio comprised between 1:14 and 1:22; heating at a temperature comprised between 50° and 70 °C, for a time comprised between 40 and 80 minutes, under stirring and obtaining a liquid phase (d) of the second extract (c) in a purified form (h), wherein the liquid phase (d) of the second extract (c), according to step H, is subjected to subsequent D and E steps.

3. Method according to claim 1, wherein the isolation step C further comprises, before step D, the steps of:
I. Loading the liquid phase (d) of the second extract (c) on a column packed with adsorption resin
J. Performing a first elution of the liquid phase (d) of the second extract (c) with water, recirculating, and discarding a first eluate (j) obtained from said first elution,
K. Performing a second elution of the liquid phase (d) of the second extract (c) with ethanol, recirculating, to an alcohol content comprised between 70° and 90° and recovering a second eluate (k) from said second elution to obtain a liquid phase (d) of the second extract (c) in a purified form, wherein the liquid phase (d) of the second extract (c), according to step K, is subjected to subsequent D and E steps.

4. Method according to claim 1 comprising, between the steps A and B, a preliminary treatment of the first extract (a) according to the following steps of:
L. Mixing the first extract (a) with water, in a weight ratio comprised between 1:10 and 1: 20; heating at a temperature comprised between 50° and 70 °C for a time comprised between 40 and 80 minutes and obtaining an intermediate extract (m) of the first extract (a),
M. Isolating, by separation, a liquid phase of said intermediate extract (m) from a solid phase (n) of said intermediate extract (m) and recovering the solid phase (n) of said intermediate extract (m) to obtain a first extract (a) in a purified form, wherein the first extract (a) according to step M is subjected to subsequent steps B, C, D and E.

5. Method according to any one of claims from 1 to 4, wherein the dry extract thereby obtained has a title higher than 5% and lower than or equal to 20%.

6. Dry extract of red rice fermented by Monascus purpureus obtained with the method according to any one of the claims from 1 to 5, having a title of total monacolins, expressed as monacolin K, higher than 5% and lower than or equal to 80%, wherein said total monacolins are 100% natural and totally obtained by fermentation of rice by Monascus purpureus.

7. Dry extract according to claim 6 having a title higher than 5% and lower than or equal to 20%.

8. Dry extract according to claim 6 or 7 for use in the treatment of hypercholesterolemia.

## Patentansprüche

1. Verfahren zur Produktion eines Trockenextrakts von rotem Reis, der mittels Monascus purpureus mit einem Titel von Gesamtmonacolinen, ausgedrückt als Monacolin K, von mehr als 5 % und weniger als oder gleich 80 % fermentiert wird, wobei die Gesamtmonacoline 100 % natürlich sind und durch Fermentierung von Reis durch Monascus purpureus erhalten werden, wobei das Verfahren die folgenden Schritte umfasst:
A. Anordnen eines ersten Trockenextrakts (a) von rotem Reis, fermentiert durch Monascus purpureus, mit einem Titel von Gesamtmonacolinen, ausgedrückt als Monacolin K, von weniger als oder gleich 3,30 %,
B. Mischen des ersten Extrakts (a) mit einem hydroalkoholischen Lösungsmittel (b) in einem Gewichtsverhältnis zwischen 1:14 und 1:22; Erwärmen bei einer Temperatur zwischen 50 °C und 70 °C für eine Zeit zwischen 40 und 80 Minuten unter Rühren und Erhalten eines zweiten Extrakts (c),
C. Isolieren, durch Trennung, einer Flüssigphase (d) des zweiten Extrakts (c) von einer Festphase des zweiten Extrakts (c),
D. Konzentrieren der Flüssigphase (d) des zweiten Extrakts (c) unter Vakuum bei einer Temperatur von weniger als 45 °C bis zum Erhalten eines weichen Extrakts (e) mit einer Resttrockenheit von mindestens 20 %,
E. Trocknen des weichen Extrakts (e) bis zum Erreichen des gewünschten Titels t und **dadurch gekennzeichnet, dass**
das alkoholische Lösungsmittel (b) ein Ethanol-Wasser-Gemisch mit einem Alkoholgehalt zwischen 30° und 96° Alkoholgrad ist.

2. Verfahren nach Anspruch 1, wobei der Isolierungsschritt C vor Schritt D die
F. Verdünnen der Flüssigphase (d) des zweiten Extrakts (c) mit Wasser und Erhalten einer Suspension (f) der Flüssigphase (d) des zweiten Extrakts (c) mit einem Alkoholgehalt zwischen 15° und 20°,
G. Isolieren, durch Trennung, einer Flüssigphase der Suspension (f) von einer Festphase (g) der Suspension (f),
H. Mischen der Festphase (g) der Suspension (f) der Flüssigphase (d) des zweiten Extrakts (c) mit dem hydroalkoholischen Lösungsmittel (b) in einem Gewichtsverhältnis zwischen 1:14 und 1:22; Erwärmen bei einer Temperatur zwischen 50 °C und 70 °C für eine Zeit zwischen 40 und 80 Minuten unter Rühren und Erhalten einer Flüssigphase (d) des zweiten Extrakts (c) in gereinigter Form (h), wobei die Flüssigphase (d) des zweiten Extrakts (c) gemäß Schritt H den nachfolgenden Schritten D und E unterzogen wird.

3. Verfahren nach Anspruch 1, wobei der Isolierungsschritt C vor Schritt D die folgenden Schritte umfasst:
I. Laden der Flüssigphase (d) des zweiten Extrakts (c) in eine Kolonne, die mit Adsorptionsharz gefüllt ist
J. Durchführen einer ersten Elution der Flüssigphase (d) des zweiten Extrakts (c) mit Wasser, Rezirkulieren und Verwerfen des ersten Eluats (j), das aus der ersten Elution erhalten wird,
K. Durchführen einer zweiten Elution der Flüssigphase (d) des zweiten Extrakts (c) mit Ethanol, Rezirkulieren, auf einen Alkoholgehalt zwischen 70° und 90° und Rückgewinnen des zweiten Eluats (k) aus der zweiten Elution, um eine Flüssigphase (d) des zweiten Extrakts (c) in einer gereinigten Form zu erhalten, wobei die Flüssigphase (d) des zweiten Extrakts (c) gemäß Schritt K nachfolgenden Schritten D und E unterzogen wird.

4. Verfahren nach Anspruch 1, das zwischen den Schritten A und B eine Vorabbehandlung des ersten Extrakts (a) gemäß den folgenden Schritten umfasst:
L. Mischen des ersten Extrakts (a) mit Wasser in einem Gewichtsverhältnis zwischen 1: 10 und 1:20; Erwärmen bei einer Temperatur zwischen 50 °C und 70 °C für eine Zeit zwischen 40 und 80 Minuten und Erhalten eines Zwischenextrakts (m) des ersten Extrakts (a),
M. Isolieren, durch Trennung, einer Flüssigphase des Zwischenextrakts (m) aus einer Festphase (n) des Zwischenextrakts (m) und Rückgewinnen der Festphase (n) des Zwischenextrakts (m), um ein erstes Extrakt (a) in einer gereinigten Form zu erhalten, wobei das erste Extrakt (a) gemäß Schritt M nachfolgenden Schritten B, C, D und E unterzogen wird.

5. Verfahren nach einem der Ansprüche 1 bis 4, wobei das dadurch erhaltene Trockenextrakt einen Titel von mehr als 5 % und weniger als oder gleich 20 % aufweist.

6. Trockenextrakt von rotem Reis, der mittels Monascus purpureus fermentiert wird, das mit dem Verfahren nach einem der Ansprüche 1 bis 5 erhalten wird und einen Titel von Gesamtmonacolinen, ausgedrückt als Monacolin K, von mehr als 5 % und weniger als oder gleich 80 % aufweist, wobei die Gesamtmonacoline 100 % natürlich sind und durch Fermentierung von Reis durch Monascus purpureus erhalten werden.

7. Trockenextrakt nach Anspruch 6, der einen Titel von mehr als 5 % und weniger als oder gleich 20 % aufweist.

8. Trockenextrakt nach Anspruch 6 oder 7 zur Verwendung bei der Behandlung von Hypercholesterinämie.

## Revendications

1. Procédé de production d'un extrait sec de riz rouge fermenté par Monascus purpureus ayant un titre de monacolines totales, exprimé en monacoline K, supérieur à 5% et inférieur ou égal à 80%, où lesdites monacolines totales sont 100% naturelles et totalement obtenues par fermentation de riz par Monascus purpureus, le procédé comprenant les étapes consistant à :
A. Disposer d'un premier extrait sec (a) de riz rouge fermenté par Monascus purpureus ayant un titre de monacolines totales, exprimé en monacoline K, inférieur ou égal à 3,30%,
B. Mélanger ledit premier extrait (a) avec un solvant hydro-alcoolique (b) dans un rapport pondéral compris entre 1:14 et 1:22; chauffer à une température comprise entre 50° et 70°C, pendant une durée comprise entre 40 et 80 minutes, sous agitation et obtenir un second extrait (c),
C. Isoler, par séparation, une phase liquide (d) dudit second extrait (c) d'une phase solide dudit second extrait (c),
D. Concentrer sous vide ladite phase liquide (d) du second extrait (c) à une température inférieure à 45°C, jusqu'à obtention d'un extrait mou (e) avec un résidu sec au moins égal à 20%,
E. Sécher ledit extrait mou (e) jusqu'à atteindre le titre t désiré, et **caractérisé en ce que**
ledit solvant alcoolique (b) est un mélange éthanol/eau ayant une teneur en alcool comprise entre 30° et 96° degrés alcooliques.

2. Procédé selon la revendication 1, où l'étape d'isolation C comprend en outre, avant l'étape D, les étapes consistant à :
F. Diluer la phase liquide (d) du second extrait (c) avec de l'eau et obtenir une suspension (f) de la phase liquide (d) du second extrait (c) avec une teneur en alcool comprise entre 15° et 20°,
G. Isoler, par séparation, une phase liquide de ladite suspension (f) d'une phase solide (g) de ladite suspension (f),
H. Mélanger la phase solide (g) de la suspension (f) de la phase liquide (d) du second extrait (c) avec le solvant hydro-alcoolique (b) dans un rapport pondéral compris entre 1:14 et 1:22 ; chauffer à une température comprise entre 50° et 70°C, pendant une durée comprise entre 40 et 80 minutes, sous agitation et obtention d'une phase liquide (d) du second extrait (c) sous une forme purifiée (h), où la phase liquide (d) du second extrait (c), selon l'étape H, est soumise aux étapes ultérieures D et E.

3. Procédé selon la revendication 1, où l'étape d'isolation C comprend en outre, avant l'étape D, les étapes consistant à :
I. Charger la phase liquide (d) du second extrait (c) sur une colonne remplie de résine d'adsorption
J. Réaliser une première élution de la phase liquide (d) du second extrait (c) avec de l'eau, recirculer et éliminer un premier éluat (j) obtenu à partir de ladite première élution,
K. Réaliser une seconde élution de la phase liquide (d) du second extrait (c) avec de l'éthanol, recirculer, à une teneur en alcool comprise entre 70° et 90° et récupérer un second éluat (k) à partir de ladite seconde élution pour obtenir une phase liquide (d) du second extrait (c) sous une forme purifiée, où la phase liquide (d) du second extrait (c), selon l'étape K, est soumise aux étapes ultérieures D et E.

4. Procédé selon la revendication 1 comprenant, entre les étapes A et B, un traitement préliminaire du premier extrait (a) selon les étapes suivantes consistant à :
L. Mélanger le premier extrait (a) avec de l'eau, dans un rapport pondéral compris entre 1: 10 et 1:20 ; chauffer à une température comprise entre 50° et 70°C pendant une durée comprise entre 40 et 80 minutes et obtenir un extrait intermédiaire (m) du premier extrait (a),
M. Isoler, par séparation, une phase liquide dudit extrait intermédiaire (m) à partir d'une phase solide (n) dudit extrait intermédiaire (m) et récupérer la phase solide (n) dudit extrait intermédiaire (m) pour obtenir un premier extrait (a) sous une forme purifiée, où le premier extrait (a) selon l'étape M est soumis aux étapes ultérieures B, C, D et E.

5. Procédé selon l'une quelconque des revendications 1 à 4, où l'extrait sec ainsi obtenu a un titre supérieur à 5% et inférieur ou égal à 20%.

6. Extrait sec de riz rouge fermenté par Monascus purpureus obtenu par le procédé selon l'une quelconque des revendications 1 à 5, ayant un titre de monacolines totales, exprimé en monacoline K, supérieur à 5% et inférieur ou égal à 80%, où lesdites monacolines totales sont 100% naturelles et totalement obtenues par fermentation de riz par Monascus purpureus.

7. Extrait sec selon la revendication 6, ayant un titre supérieur à 5% et inférieur ou égal à 20%.

8. Extrait sec selon la revendication 6 ou 7 destiné à être utilisé dans le traitement de l'hypercholestérolémie.
